# FASCICULE DE BREVET EUROPEEN

(11) **EP 1 566 165 B1**
(45) Date de publication et mention de la délivrance du brevet: **14.08.2013**
(21) Numéro de dépôt: 05290290.5
(22) Date de dépôt: 09.02.2005
(51) Int. Cl.: A61Q 5/06, A61K 8/34, A61K 8/81, A61K 8/86, A61K 8/894

(54) **Utilisation d'un composé non-ionique et d'un polymère fixant anionique pour la mise en forme des fibres kératiniques avec application d'un moyen chauffant**
Verwendung einer nichtionischen Verbindung und eines anionischen Festigungspolymers zur Verformung von Keratinfasern unter Verwendung von Heizmitteln
Use of non-ionic compound and an anionic fixative polymer for the shaping of keratin fibers by using heating means

(30) Priorité: 12.02.2004 FR 0401401
(43) Date de publication de la demande: 24.08.2005
(73) Titulaire: L'Oréal, 75008 Paris (FR)
(72) Inventeur: Laurent, Ludvine, 92400 Courbevoie (FR); Cochonneau, Laurent, 72220 Laigne-en-Belin (FR); Bebot, Cécile, 92110 Clichy (FR)
(74) Mandataire: Casalonga, Axel

(56) Documents cités:
- EP-A- 0 524 346
- EP-A- 0 791 351
- EP-A- 1 348 420
- WO-A-00/40628
- WO-A-00/69398
- US-A- 3 433 868

## Description

La présente invention a pour objet l'utilisation d'au moins un composé non-ionique choisi parmi les alcools gras éthoxylés par 2 à 10 groupements OEthyle, les polyols et les silicones linéaires, cycliques, ramifiées ou non ramifiées, volatiles ou non volatiles, choisies parmi les polyorganosiloxanes comportant des groupements polyéthyléneoxy et/ou polypropyléneoxy pour diminuer les résidues inesthétiques des polymères fixants anioniques lors de la mise en contact de ces polymères avec un moyen chauffant, notamment pour la mise en forme des fibres kératinique.

L'utilisation de moyens chauffants pour la mise en forme des fibres kératiniques tels que les fers à lisser, à boucler, à gaufrer est connue. La Demanderesse a également utilisé ces moyens chauffants en association avec des compositions contenant notamment des polymères, éventuellement anioniques comme cela est décrit dans la demande française de numéro de dépôt FR 03 05636. Les résultats obtenus peuvent s'accompagner d'une formation sur les cheveux de résidus inesthétiques sous la forme de particules blanchâtres.

Par ailleurs, l'utilisation de polymères bloc/branchés comprenant des blocs hydrophiles et hydrophobes particuliers dans des compositions de coiffage est connue dans la demande de brevet WO 00/40628.

Le but de la présente invention est de proposer un procédé de mise en forme des fibres kératiniques qui ne génère pas de tels résidus inesthétiques.

De manière avantageuse et inattendue, la Demanderesse vient maintenant de découvrir que ce problème peut être résolu par la mise en oeuvre d'un procédé comprenant l'application sur les fibres kératiniques d'une composition à base d'au moins un polymère fixant anionique particulier et d'au moins un composé non-ionique particulier et de la mise en contact des fibres kératiniques, de manière séquentielle ou simultanée, avec un moyen chauffant apte à la mise en forme des fibres kératiniques.

Le procédé selon l'invention est de mise en oeuvre facile : il permet une mise en forme aisée de la coiffure désirée, et conduit à un bon niveau de fixation des fibres kératiniques qui se maintient tout au long de la journée voire sur plusieurs jours, l'effet obtenu présente également une bonne tenue à l'humidité et s'élimine facilement au shampooing ; de plus ce procédé permet d'éviter la formation sur les fibres kératiniques de particules blanchâtres inesthétiques.

Ce procédé permet aussi de conférer aux cheveux de bonnes propriétés cosmétiques telles que douceur, démêlage facile, brillance.

D'autres caractéristiques, aspects, objets et avantages de la présente invention apparaîtront encore plus clairement à la lecture de la description et des exemples qui suivent.

Un premier objet de la présente demande concerne donc l'utilisation d'au moins un composé non-ionique particuliers, c'est-à-dire choisi parmi les alcools gras, éventuellement éthoxylés, les polyols et les silicones linéaires, cycliques, ramifiées ou non ramifiées, volatiles ou non volatiles, choisies parmi les polyorganosiloxanes comportant des groupements polyéthyléneoxy et/ou polypropyléneoxy pour diminuer les résidus inesthétiques de polymères fixants anionques particuliers lors de la mise en contact de ces polymères avec un moyen chauffant, lesdits polymères fixants anioniques particuliers, c'est-à-dire étant choisis parmi :
- les polymères comportant des groupements dérivés d'acide carboxylique, sulfonique ou phosphorique et qui ont une masse moléculaire en nombre comprise entre environ 500 et 5 000 000, les polymères comportant des groupements dérivés d'acide carboxylique étant choisi parmi :
   A) Les homo- ou copolymères d'acide acrylique ou méthacrylique ou leurs sels, les sels de sodium des acides polyhydroxycarboxyliques ;
   B) Les copolymères d'acide acrylique ou méthacrylique avec un monomère monoéthylénique tel que l'éthylène, le styrène, les esters vinyliques, éventuellement greffés sur un polyalkylène-glycol et éventuellement réticulés;
   C) Les copolymères d'acide crotonique;
   D) Les copolymères d'acides ou d'anhydrides carboxyliques monoinsaturés en C₄-C₈ choisis parmi : - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées; - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées ;
   E) Les polyacrylamides comportant des groupements carboxylates ;
   F) les polymères comportant des groupements dérivés d'acide sulfoniques étant choisi parmi les homopolymères et copolymères comportant des motifs vinylsulfonique, styrène-sulfonique, naphtalène-sulfonique ou acrylamido-alkylsulfonique
- les polymères fixants anioniques de type siliconés greffés, comprenant une partie polysiloxane et une partie constituée d'une chaîne organique non siliconée, l'une des deux parties constituant la chaîne principale du polymère et l'autre étant greffée sur ladite chaîne principale ;
- les polyuréthanes anioniques fonctionnalisés ou non, siliconés ou non ;
- les copolymères à bloc ramifié comprenant, comme monomères principaux, au moins un acrylate d'alkyle en C1-20 et/ou au moins un N-mono, ou N,N-di(alkyle en C2-12)(meth)acrylamide, et de l'acide acrylique et/ou de l'acide méthacrylique.

Un second objet de la présente demande est donc une utilisation de la revendication 1 pour la mise en forme des fibres kératiniques comprenant les deux étapes principales suivantes :
- appliquer sur les fibres kératiniques une composition cosmétique comprenant au moins un polymère fixant anionique particulier et au moins un composé non-ionique particulier; ce polymère fixant et ce composé tels que définis à la revendication 1,
- et, après un éventuel temps de pause, mettre les fibres kératiniques en contact avec un moyen chauffant apte à mettre en forme lesdites fibres kératiniques.

De préférence, une composition particulière utilisée selon l'invention contient au moins un polymère fixant anionique particulier et au moins un composé non-ionique particulier, le rapport pondéral polymère(s) fixant(s) anionique(s) particulier(s)/ composé(s) non-ionique(s) particulier(s) étant supérieur à 1

Par « procédé de mise en forme des matière kératiniques », on entend tout procédé destiné à modifier l'aspect initial de la coiffure, il peut donc s'agir d'un procédé de frisage ou de gaufrage des cheveux ou encore d'un procédé de défrisage ou de lissage des cheveux.

De préférence, les polymères particuliers des compositions particulières de l'invention sont les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique.

Lorsque la composition cosmétique est appliquée sur les fibres kératiniques avant l'étape d'application du moyen chauffant, un temps de pause compris entre 30 secondes et 10 minutes peut être effectué entre ces deux étapes principales.

L'étape de mise en contact des fibres kératiniques avec un moyen chauffant peut être effectuée au moyen de tout dispositif chauffant apte à mettre en forme lesdites fibres kératiniques. Ces appareils permettent généralement de chauffer les fibres kératiniques à une température comprise entre 50°C et 250°C, de préférence entre 100°C et 230°C.

Classiquement, cette étape est une étape de frisage ou de gaufrage des cheveux ou encore de défrisage ou de lissage des cheveux, elle est mise en oeuvre au moyen de tout dispositif approprié tel que les fers à lisser, à boucler, à gaufrer.

L'application du moyen chauffant peut se faire par touches successives ou en glissant l'appareil le long des fibres.

Selon une variante de l'invention, le moyen chauffant, et en particuliers les fers, possède un réservoir permettant la délivrance directe de la composition sur les fibres.

L'étape consistant à appliquer une composition cosmétique est effectuée au moyen d'une composition comprenant au moins un polymère fixant anionique particulier et au moins un composé non-ionique particulier.

De préférence, le rapport pondéral polymère(s) fixant(s) anioniques particulier(s)/composé(s) non-ionique(s) particulier(s) est supérieur ou égal à 1.

Par « polymère fixant », on entend, au sens de la présente demande, tout polymère apte à conférer une forme à la chevelure ou à modifier sa forme originelle.

Les polymères fixants anioniques particuliers sont détaillés dans les passages qui suivent.

A titre de polymère fixant anionique particulier utilisable dans la composition cosmétique mise en oeuvre dans le procédé selon l'invention, on compte les polymères comportant des groupements dérivés d'acide carboxylique, sulfonique ou phosphorique et qui ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 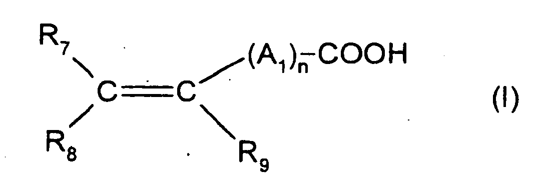 dans laquelle n est un nombre entier de 0 à 10, A, désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques utilisables selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n^{o} 1 222 944 et la demande allemande n^{o} 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ® AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan® 500 et Flexan® 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® ES 425 par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.

Selon l'invention, on peut également utiliser des polymères fixants anioniques de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 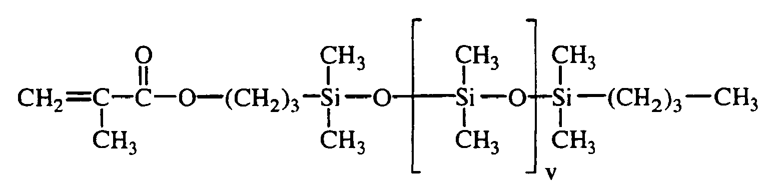
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle).

Comme autre type de polymères fixants siliconés anioniques, on peut citer le produit Luviflex® Silk commercialisé par la société BASF.

On peut également utiliser comme polymères fixants, des polyuréthanes anioniques fonctionnalisés ou non, siliconés ou non.

Les polyuréthanes particulièrement visés sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

Parmi les polymères fixants anioniques utilisables au sens de la présente invention, on compte aussi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique.

Ces copolymères séquencés (ou à blocs) ramifiés présentent une structure constituée de blocs hydrophobes sur lesquels sont fixés, notamment par l'intermédiaire de motifs bifonctionnels, un certain nombre de blocs plus hydrophiles. Ces copolymères présentent au moins deux températures de transition vitreuse.

Ces copolymères présentent la composition suivante :
de 26 à 36 % en moles d'acide acrylique
de 27,5 à 30,5 % en moles d'acrylate de n-butyle
de 33,3 à 45,3 % en moles d'acide métacrylique
de 0,48 à 0,92 % en moles de méthacrylate d'allyle

Les blocs les plus hydrophobes ont un poids moléculaire de 10 000 à 100 000 et les blocs les plus hydrophiles ont un poids moléculaire de 1000 à 100 000 daltons.

Les polymères filmogènes fixants ci-dessus sont sous forme anionique, c'est-à-dire qu'ils sont transformés en sels par neutralisation partielle ou totale des groupes acide (méth)acrylique. On peut citer à titre d'exemple d'agent de neutralisation préféré le 2-amino-2-méthyl-1-propanol ou l'hydroxyde de sodium.

Ils sont notamment décrits dans la demande de brevet WO 00/40628 et commercialisés par exemple sous les dénominations EX-SDR-26® et EX-SDR-45® par la société GOODRICH.

Les polymère(s) fixant(s) particulier(s) sont de préférence utilisés dans les compositions selon la présente invention en une concentration allant de 0,1 à 20%, de préférence de 0,5 à 10% et de manière encore plus préférée de 0,6 à 5% en poids par rapport au poids total de la composition.

Dans les compositions particulières selon l'invention, le ou les polymères fixants anioniques particuliers choisis parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique sont avantageusement utilisés dans une concentration allant de 0,6 à 5% en poids par rapport au poids total de la composition.

A titre de composé non-ionique utilisable dans la composition cosmétique mise en oeuvre dans le procédé selon l'invention, on compte les alcools gras éventuellement ethoxylés, les polyols, et les silicones.

Au sens de la présente demande, on entend par « alcools gras », les alcools linéaires ou ramifiés, de préférence linéaires, éventuellement insaturés comprenant de 4 à 30, de préférence de 8 à 22 et de manière encore plus préférée de 12 à 22 atomes de carbone.

Ces alcools gras peuvent être ethoxylés, ils comprennent alors de 2 à 10 groupement OEthyle. De préférence les alcools gras ethoxylés utilisables selon l'invention présentent une HLB>5, ces alcools gras sont utilisables de manière préférée dans les compositions particulières selon la présente demande.

Les polyols utilisables dans le cadre de la présente invention sont de préférence des polyols en C₃-C₁₀, comprenant de 2 à 5 groupements OH. Ils sont choisis en particulier dans le groupe formé par le glycérol, le glycol, le propylèneglycol, le dipropylèneglycol, le butylèneglycol et le butylediglycol.

Les silicones utilisables dans le cadre de la présente invention peuvent être linéaires, cycliques, ramifiées ou non ramifiées, volatiles ou non volatiles. Elles sont choisies parmi les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C12)-méthicone-copolyol commercialisée par la société DOW CORNING sous la dénomination Q2 5200. Ces silicones sont particulièrement préférées dans le cadre la présente invention .

La concentration en composé(s) non-ionique(s) particulier(s) utilisée dans les compositions selon la présente invention va de 0,1 à 20%, de préférence de 0,5 à 10% et de manière encore plus préférée de 0,6 à 5% en poids par rapport au poids total de la composition.

De manière particulièrement préférée, la composition cosmétique utilisée dans le procédé selon la présente invention comprend un polymère fixant anionique de type copolymère d'AMP Acrylate/methacrylate d'allyle (ex Fixate G100 de la société NOVEON) en une concentration allant de 0,6% à 5 % en poids par rapport au poids total de la composition, un composé non-ionique particulier choisi parmi les polyols en C₃-C₁₀, les alcools gras ethoxylés de HLB >5 et les silicones copolyols en une concentration allant de 0,1% à 5 % en poids par rapport au poids total de la composition, cette composition étant telle que le rapport polymère fixant anionique particulier/composé(s) non-ionique(s) particulier(s), est supérieur à 1.

La composition selon l'invention ou la composition particulière selon l'invention peuvent contenir en outre au moins un adjuvant choisi parmi les agents tensio-actifs non-ioniques, anioniques, cationiques et amphotères, les polymères non-ioniques, anioniques, cationiques et amphotères additionnels (autres que les polymères fixants anioniques décrits ci-dessus), les céramides et pseudo-céramides, les vitamines et pro-vitamines dont le panthénol, les filtres solaires hydrosolubles et liposolubles, liquides ou solides, les composés solides tels que les pigments, les agents nacrants ou opacifiants, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les solvants, les agents de pénétration, les parfums, les tampons, les agents dispersants, les agents de conditionnement, les agents opacifiants et les agents conservateurs.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, hydroxylés ou non, tels que l'éthanol et l'isopropanol ; les éthers de polyols comme le 2-butoxyéthanol, le propylèneglycol, le monométhyléther de propytèneglycol, le monoéthyléther et le monométhyléther du diéthylèneglycol, les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les exemples qui suivent servent à illustrer l'invention sans toutefois présenter un caractère limitatif.

### EXEMPLES

On prépare les formulations des exemples suivants :

Fixate G100 (Noveon) est un copolymère d'acrylates AMP/méthacrylate d'allyle.

| Formulation 1 | |
|---|---|
| STEARETH-21 | 0,6% |
| Alcool | 40% |
| Propyléne glycol | 1,4% |
| Fixate G100 (Noveon) | 3,2% M.A. |
| AMP | pH |
| Eau désionisée | Qs100 |
| parfum | qsp |

| Formulation 2 | |
|---|---|
| DIMETHICONE POLYOL (DC 1248) | 1% |
| Alcool | 40% |
| Fixate G100 (Noveon) | 3,15% M.A. |
| AMP | pH |
| Eau désionisée | Qs100 |
| parfum | qsp |

Chacune de ces formulations est appliquée sur un panel de modèles et sur des mèches de cheveux naturels.

Après un temps de pause d'environ trente secondes, on applique un fer plat ou rond chauffant à 200°C apte à mettre en forme ces cheveux.

Deux séries d'essais ont été menées :

La première série a consisté à lisser chaque mèche de cheveux traitées avec les formulations 1, et 2 au moyen d'un fer plat (Techniliss Ioni Ceramic PNC 228 VELECTA) en effectuant plusieurs passages en mouvement continu de la racine à la pointe.

La deuxième série a consisté en une mise en forme des cheveux au fer à boucler.

On observe une bonne tenue de la coiffure obtenue, les cheveux sont doux, les mèches sont lisses et brillantes, en outre aucun dépôt de résidus blanchâtres, inesthétiques n'est observé.

## Revendications

1. Utilisation d'au moins un composé non-ionique choisi parmi les alcools gras, éthoxylés par 2 à 10 groupements OEthyle, les polyols et les silicones linéaires, cycliques, ramifiées ou non ramifiées, volatiles ou non volatiles, choisies parmi les polyorganosiloxanes comportant des groupements polyéthyléneoxy et/ou polypropyléneoxy, pour diminuer les résidus inesthétiques de polymères fixants anioniques lors de la mise en contact de ces polymères avec un moyen chauffant, lesdits polymères anioniques étant choisis parmi :
• les polymères comportant des groupements dérivés d'acide carboxylique, sulfonique ou phosphorique et qui ont une masse moléculaire en nombre comprise entre environ 500 et 5 000 000, les polymères comportant des groupements dérivés d'acide carboxylique étant choisi parmi :
A) Les homo- ou copolymères d'acide acrylique ou méthacrylique ou leurs sels, les sels de sodium des acides polyhydroxycarboxyliques ;
B) Les copolymères d'acide acrylique ou méthacrylique avec un monomère monoéthylénique tel que l'éthylène, le styrène, les esters vinyliques, éventuellement greffés sur un polyalkylène-glycol et éventuellement réticulés ;
C) Les copolymères d'acide crotonique;
D) Les copolymères d'acides ou d'anhydrides carboxyliques monoinsaturés en C₄-C₈ choisis parmi : - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées; - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolyméres étant éventuellement monoestérifiées ou monoamidifiées;
E) Les polyacrylamides comportant des groupements carboxylates ;
F) les polymères comportant des groupements dérivés d'acide sulfoniques étant choisi parmi les homopolymères et copolymères comportant des motifs vinylsulfonique, styrène-sulfonique, naphtalène-snlfonique ou acrylamido-alkylsulfonique
• les polymères fixants anioniques de type siliconés greffés, comprenant une partie polysiloxane et une partie constituée d'une chaîne organique non siliconée, l'une des deux parties constituant la chaîne principale du polymère et l'autre étant greffée sur ladite chaîne principale ;
• les polyuréthanes anioniques fonctionnalisés ou non, siliconés ou non ;
• les copolymères à bloc ramifié comprenant, comme monomères principaux, au moins un acrylate d'alkyle en C1-20 et/ou au moins un N-mono, ou N,N-di(alkyle en C2-12)(meth)acrylamide, et de l'acide acrylique et/ou de l'acide méthacrylique.

2. Utilisation selon la revendication 1 pour la mise en forme des fibres kératiniques, de préférence les fibres kératiniques humaines, en particulier les cheveux, comprenant les deux étapes principales suivantes :
appliquer sur les fibres keratiniques, de préférence les fibres keratiniques humaines, en particulier les cheveux, une composition cosmétique comprenant au moins un polymère fixant anionique défini à la revendication 1, et au moins un composé non-ionique défini à la revendication 1
- et, après un éventuel temps de pause, mettre les fibres kératiniques en contact avec un moyen chauffant apte à mettre en forme lesdites fibres kératiniques..

3. Utilisation selon la revendication 2, **caractérisée en ce que** entre les deux étapes principales, un temps de pause compris entre 30 secondes et 10 minutes est effectué.

4. Utilisation selon la revendication 2 ou 3, **caractérisée en ce que** l'étape de mise en contact des fibres kératiniques avec un moyen chauffant est effectuée à une température comprise entre 50°C et 250°C, de préférence entre 100°C et 230°C.

5. Utilisation selon l'une quelconque des revendications 1 à 4, **caractérisée en ce que** le polymère fixant anionique est un copolymère à bloc ramifié comprenant, comme monomères, de l'acrylate de n-butyle, de l'acide acrylique, de l'acide méthacrylique et du méthacrylate d'allyle.

6. Utilisation selon l'une quelconque des revendication 2 à 5, **caractérisée en ce que** la concentration en polymère(s) fixant(s) anionique(s) de la composition cosmétique va de 0,1 à 20%, de préférence de 0,5 à 10% et de manière encore plus préférée de 0,6 à 5% en poids par rapport au poids total de la composition.

7. Utilisation selon l'une quelconque des revendications 1 à 6, **caractérisée en ce que** le composé non-ionique est un alcool gras comprenant de 4 à 30, de préférence de 8 à 22 et de manière encore plus préférée de 12 à 22 atomes de carbone.

8. Utilisation selon la revendication 7, **caractérisée en ce que** l'alcool gras est un alcool gras ethoxylé de HLB>5.

9. Utilisation selon l'une quelconque des revendications 1 à 8, **caractérisée en ce que** le composé non-ionique est un polyol en C₃-C₁₀, comprenant de 2 à 5 groupements OH.

10. Utilisation selon la revendication 8, **caractérisée en ce que** polyol est choisi dans le groupe formé par le glycérol, le glycol, le propylèneglycol, le dipropylèneglycol, le butylèneglycol et le butyldiglycol.

11. Utilisation selon l'une quelconque des revendications 1 à 10, caractérisée ce que le composé non-ionique est une silicone linéaire, cyclique, ramifiée ou non ramifiée, volatile ou non volatile choisie parmi les polyorganosiloxanes comportant des groupements polyéthyléneoxy et/ou polypropyléneoxy.

12. Utilisation selon la revendication 11, **caractérisée en ce que** la silicone est un polyorganosiloxane comportant des groupements polyéthylèneoxy et/ou polypropylèneoxy comportant des groupements alkyle en C6-C24.

13. Utilisation selon l'une quelconque des revendications 2 à 12, **caractérisée en ce que** la concentration en composé(s) non-ionique(s) de la composition cosmétique va de 0,1 à 20%, de préférence de 0,5 à 10% et de manière encore plus préférée de 0,6 à 5% en poids par rapport au poids total de la composition.

14. Utilisation selon l'une quelconque des revendications 2 à 13, **caractérisée en ce que** le rapport pondéral polymère(s) fixant(s) anionique(s)/ composé(s) non-ionique(s) dans la composition est supérieur à 1.

15. Utilisation selon l'une quelconque des revendications 2 à 14, **caractérisée en ce que** la composition cosmétique comprend au moins un adjuvant choisi parmi les agents tensio-actifs non-ioniques, anioniques, cationiques et amphotères, les polymères non-ioniques, anioniques, cationiques et amphotères additionnels, les céramides et pseudo-céramides, les vitamines et pro-vitamines dont le panthénol, les filtres solaires hydrosolubles et liposolubles, liquides ou solides, les composés solides tels que les pigments, les agents nacrants ou opacifiants, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les solvants, les agents de pénétration, les parfums, les tampons, les agents dispersants, les agents de conditionnement, les agents opacifiants et les agents conservateurs.

## Patentansprüche

1. Verwendung mindestens einer nichtionischen Verbindung, ausgewählt aus durch 2 bis 10 O-Ethyl-Gruppen ethoxylierten Fettalkoholen, Polyolen und flüchtigen oder nicht flüchtigen, linearen, cyclischen, verzweigten oder unverzweigten Silikonen, ausgewählt aus Polyorganosiloxanen mit Polyethylenoxy- und/oder Polypropylenoxygruppen, zur Verringerung der unästhetischen Rückstände von anionischen fixierenden Polymeren beim Inberührungbringen dieser Polymere mit einem Heizmittel, wobei die anionischen Polymere ausgewählt sind aus:
• Polymeren mit Gruppen, die sich von einer Carbonsäure, Sulfonsäure oder Phosphorsäure ableiten, und einem zahlenmittleren Molekulargewicht zwischen ungefähr 500 und 5.000.000, wobei die Polymere mit Gruppen, die sich von einer Carbonsäure ableiten, ausgewählt sind aus:
A) Homo- oder Copolymeren von Acryl- oder Methacrylsäure oder Salzen davon, wie Natriumsalzen von Polyhydroxycarbonsäuren;
B) Copolymeren von Acryl- oder Methacrylsäure mit einem monoethylenischen Monomer wie Ethylen, Styrol, Vinylestern, gegebenenfalls auf ein Polyalkylenglykol aufgepfropft und gegebenenfalls vernetzt;
C) Copolymeren von Crotonsäure;
D) Copolymeren von einfach ungesättigten C₄-C₈-Carbonsäuren oder -Carbonsäureanhydriden, ausgewählt aus: - Copolymeren, umfassend (i) Maleinsäure, Fumarsäure, Itaconsäure, Maleinsäureanhydrid, Fumarsäureanhydrid und/oder Itaconsäureanhydrid und (ii) mindestens einem Monomer, das aus Vinylestern, Vinylethern, Vinylhalogeniden, Phenylvinylderivaten, Acrylsäure und deren Estern ausgewählt ist, wobei die Anhydridfunktionen dieser Copolymere gegebenenfalls einfach verestert oder einfach amidiert sind; - Copolymeren, umfassend (i) Maleinsäure, Citraconsäure, Itaconsäure, Maleinsäureanhydrid, Citraconsäureanhydrid und/oder Itaconsäureanhydrid und (ii) ein oder mehrere Monomere, die aus Allyl- oder Methallylestern, die gegebenenfalls ein oder mehrere Acrylamid-, Methacrylamid-, α-Olefin-, Acryl- oder Methacrylester-, Acryl- oder Methacrylsäure- oder Vinylpyrrolidingruppen in ihrer Kette umfassen, ausgewählt sind, wobei die Anhydridfunktionen dieser Copolymere gegebenenfalls einfach verestert oder einfach amidiert sind;
E) Polyacrylamiden mit Carboxylatgruppen;
F) Polymeren mit Gruppen, die sich von einer Sulfonsäure ableiten, ausgewählt aus Homopolymeren und Copolymeren mit Vinylsulfonsäure-, Styrolsulfonsäure-, Naphthalinsulfonsäure- oder Acrylamidoalkylsulfonsäure-Einheiten;
• anionischen fixierenden Polymeren vom Typ gepfropftes Silikon mit einem Polysiloxanteil und einem Teil, der aus einer organischen Nichtsilikonkette besteht, wobei einer der beiden Teile die Hauptkette des Polymers bildet und der andere auf die Hauptkette aufgepfropft ist;
• anionischen Polyurethanen, die gegebenenfalls funktionalisiert sind und gegebenenfalls silikoniert sind;
• verzweigte Blockcopolymere, die als Hauptmonomere mindestens ein C1-20-Alkylacrylat und/oder mindestens ein N-Mono- oder N,N-Di(C2-12-alkyl)(meth)acrylamid, Acrylsäure und/oder Methacrylsäure umfassen.

2. Verwendung nach Anspruch 1 zum Formen von Keratinfasern, vorzugsweise menschlichen Keratinfasern, insbesondere Haaren, das die folgenden beiden Hauptschritte umfasst:
- Aufbringen einer kosmetischen Zusammensetzung, die mindestens ein anionisches fixierendes Polymer gemäß Anspruch 1 und mindestens eine nichtionische Verbindung gemäß Anspruch 1 umfasst, auf die Keratinfasern, vorzugsweise menschlichen Keratinfasern, insbesondere Haare,
- und, gegebenenfalls nach einer Einwirkungszeit, Inberührungbringen der Keratinfasern mit einem Heizmittel, das zum Formen der Keratinfasern geeignet ist.

3. Verwendung nach Anspruch 2, **dadurch gekennzeichnet, dass** zwischen den beiden Hauptschritten eine Einwirkungszeit zwischen 30 Sekunden und 10 Minuten durchgeführt wird.

4. Verwendung nach Anspruch 2 oder 3, **dadurch gekennzeichnet, dass** der Schritt des Inberührungbringens der Keratinfasern mit einem Heizmittel bei einer Temperatur zwischen 50°C und 250°C, vorzugsweise zwischen 100°C und 230°C, durchgeführt wird.

5. Verwendung nach einem der Ansprüche 1 bis 4, **dadurch gekennzeichnet, dass** es sich bei dem anionischen fixierenden Polymer um ein verzweigtes Blockcopolymer, das als Monomere n-Butylacrylat, Acrylsäure, Methacrylsäure und Allylmethacrylat umfasst, handelt.

6. Verwendung nach einem der Ansprüche 2 bis 5, **dadurch gekennzeichnet, dass** die Konzentration an anionischem fixierendem Polymer bzw. anionischen fixierenden Polymeren der kosmetischen Zusammensetzung 0,1 bis 20 Gew.-%, vorzugsweise 0,5 bis 10 Gew.-% und noch weiter bevorzugt 0,6 bis 5 Gew.-%, bezogen auf das Gesamtgewicht der Zusammensetzung, beträgt.

7. Verwendung nach einem der Ansprüche 1 bis 6, **dadurch gekennzeichnet, dass** es sich bei der nichtionischen Verbindung um einen Fettalkohol mit 4 bis 30, bzw. 8 bis 22 und noch weiter bevorzugt 12 bis 22 Kohlenstoffatomen handelt.

8. Verwendung nach Anspruch 7, **dadurch gekennzeichnet, dass** es sich bei dem Fettalkohol um einen ethoxylierten Fettalkohol mit HLB>5 handelt.

9. Verwendung nach einem der Ansprüche 1 bis 8, **dadurch gekennzeichnet, dass** es sich bei der nichtionischen Verbindung um ein C₃-C₁₀-Polyol mit 2 bis 5 OH-Gruppen handelt.

10. Verwendung nach Anspruch 8, **dadurch gekennzeichnet, dass** das Polyol aus der Gruppe bestehend aus Glycerin, Glykol, Propylenglykol, Dipropylenglykol, Butylenglykol und Butyldiglykol ausgewählt ist.

11. Verwendung nach einem der Ansprüche 1 bis 10, **dadurch gekennzeichnet, dass** es sich bei der nichtionischen Verbindung um ein flüchtiges oder nichtflüchtiges, lineares, cyclisches, verzweigtes oder unverzweigtes Silikon, das aus Polyorganosiloxanen mit Polyethylenoxy- und/oder Polypropylenoxygruppen ausgewählt ist, handelt.

12. Verwendung nach Anspruch 11, **dadurch gekennzeichnet, dass** es sich bei dem Silikon um ein Polyorganosiloxan mit Polyethylenoxy- und/oder Polypropylenoxygruppen mit C6-C24-Alkylgruppen handelt.

13. Verwendung nach einem der Ansprüche 2 bis 12, **dadurch gekennzeichnet, dass** die Konzentration an nichtionischer Verbindung bzw. nichtionischen Verbindungen der kosmetischen Zusammensetzung 0,1 bis 20 Gew.-%, vorzugsweise 0,5 bis 10 Gew.-% und noch weiter bevorzugt 0,6 bis 5 Gew.-%, bezogen auf das Gesamtgewicht der Zusammensetzung, beträgt.

14. Verwendung nach einem der Ansprüche 2 bis 13, **dadurch gekennzeichnet, dass** das Gewichtsverhältnis von anionischem fixierendem Polymer bzw. anionischen fixierenden Polymeren zu nichtionischer Verbindung bzw. nichtionischen Verbindungen in der Zusammensetzung größer als 1 ist.

15. Verwendung nach einem der Ansprüche 2 bis 14, **dadurch gekennzeichnet, dass** die kosmetische Zusammensetzung mindestens einen Hilfsstoff, der aus nichtionischen, anionischen, kationischen und amphoteren Tensiden, zusätzlichen nichtionischen, anionischen, kationischen und amphoteren Polymeren, Ceramiden und Pseudoceramiden, Vitaminen und Provitaminen, darunter Panthenol, festen oder flüssigen, wasserlöslichen und fettlöslichen Lichtschutzmitteln, festen Verbindungen wie Pigmenten, Perlglanz- oder Trübungsmitteln, Farbmitteln, Sequestriermitteln, Weichmachern, Solubilisierungsmitteln, Ansäuerungsmitteln, Alkalinisierungsmitteln, Neutralisationsmitteln, anorganischen und organischen Verdickungsmitteln, Antioxidantien, Hydroxysäuren, Lösungsmitteln, Penetriermitteln, Duftstoffen, Puffersubstanzen, Dispergiermitteln, Konditionierungsmitteln, Trübungsmitteln und Konservierungsstoffen ausgewählt ist, umfasst.

## Claims

1. Use of at least one non-ionic compound chosen from fatty alcohols ethoxylated by 2 to 10 OEthyl groups, polyols and linear, cyclic, branched or unbranched and volatile or non-volatile silicones chosen from polyorganosiloxanes comprising polyethyleneoxy and/or polypropyleneoxy groups, in order to reduce the unsightly residues of anionic fixative polymers when these polymers are brought into contact with a heating means, the said anionic polymers being chosen from:
• polymers comprising groups derived from carboxylic, sulfonic or phosphoric acid and which have a number-average molecular weight of between approximately 500 and 5 000 000, the polymers comprising groups derived from carboxylic acid being chosen from:
A) homo- or copolymers of acrylic or methacrylic acid or their salts, or sodium salts of polyhydroxycarboxylic acids;
B) copolymers of acrylic or methacrylic acid with a monoethylenic monomer, such as ethylene, styrene or vinyl esters, optionally grafted to a polyalkylene glycol and optionally crosslinked;
C) crotonic acid copolymers;
D) copolymers of monounsaturated C₄-C₈ carboxylic acids or anhydrides chosen from: - copolymers comprising (i) one or more maleic, fumaric or itaconic acids or anhydrides and (ii) at least one monomer chosen from vinyl esters, vinyl ethers, vinyl halides, phenylvinyl derivatives, or acrylic acid and its esters, the anhydride functional groups of these copolymers optionally being monoesterified or monoamidated; - copolymers comprising (i) one or more maleic, citraconic or itaconic anhydride units and (ii) one or more monomers chosen from allyl or methallyl esters optionally comprising one or more acrylamide, methacrylamide, α-olefin, acrylic or methacrylic ester, acrylic or methacrylic acid or vinylpyrrolidone groups in their chain, the anhydride functional groups of these copolymers optionally being monoesterified or monoamidated;
E) polyacrylamides comprising carboxylate groups;
F) the polymers comprising groups derived from sulfonic acids being chosen from homopolymers and copolymers comprising vinylsulfonic, styrenesulfonic, naphthalenesulfonic or acrylamido-alkylsulfonic units;
• anionic fixative polymers of grafted silicone type, comprising a polysiloxane part and a part consisting of a non-silicone organic chain, one of the two parts constituting the main chain of the polymer and the other being grafted to the said main chain;
• functionalized or non-functionalized and silicone or non-silicone anionic polyurethanes;
• branched block copolymers comprising, as main monomers, at least one C₁₋₂₀ alkyl acrylate and/or at least one N-mono(C₂₋₁₂ alkyl)- or N,N-di(C₂₋₁₂ alkyl)(meth)acrylamide, and acrylic acid and/or methacrylic acid.

2. Use according to Claim 1 for the shaping of keratinous fibres, preferably human keratinous fibres, in particular the hair, comprising the following two main stages:
- applying to keratinous fibres, preferably human keratinous fibres, in particular the hair, a cosmetic composition comprising at least one anionic fixative polymer defined in Claim 1 and at least one non-ionic compound defined in Claim 1,
- and, after an optional leave-in time, bringing the keratinous fibres into contact with a heating means capable of shaping the said keratinous fibres.

3. Use according to Claim 2, **characterized in that** a leave-in time of within 30 seconds and 10 minutes is effected between the two main stages.

4. Use according to Claim 2 or 3, **characterized in that** the stage of bringing the keratinous fibres into contact with a heating means is carried out at a temperature of between 50°C and 250°C, preferably between 100°C and 230°C.

5. Use according to any one of Claims 1 to 4, **characterized in that** the anionic fixative polymer is a branched block copolymer comprising, as monomers, n-butyl acrylate, acrylic acid, methacrylic acid and allyl methacrylate.

6. Use according to any one of Claims 2 to 5, **characterized in that** the concentration of anionic fixative polymer(s) of the cosmetic composition ranges from 0.1% to 20% by weight, preferably from 0.5% to 10% by weight and more preferably still from 0.6% to 5% by weight, with respect to the total weight of the composition.

7. Use according to any one of Claims 1 to 6, **characterized in that** the non-ionic compound is a fatty alcohol comprising from 4 to 30, preferably from 8 to 22 and more preferably still from 12 to 22 carbon atoms.

8. Use according to Claim 7, **characterized in that** the fatty alcohol is an ethoxylated fatty alcohol with an HLB>5.

9. Use according to any one of Claims 1 to 8, **characterized in that** the non-ionic compound is a C₃-C₁₀ polyol comprising from 2 to 5 OH groups.

10. Use according to Claim 8, **characterized in that** the polyol is chosen from the group formed by glycerol, glycol, propylene glycol, dipropylene glycol, butylene glycol and butyl diglycol.

11. Use according to any one of Claims 1 to 10, **characterized in that** the non-ionic compound is a linear, cyclic, branched or unbranched and volatile or non-volatile silicone chosen from polyorganosiloxanes comprising polyethyleneoxy and/or polypropyleneoxy groups.

12. Use according to Claim 11, **characterized in that** the silicone is a polyorganosiloxane comprising polyethyleneoxy and/or polypropyleneoxy groups comprising C₆-C₂₄ alkyl groups.

13. Use according to any one of Claims 2 to 12, **characterized in that** the concentration of non-ionic compound(s) of the cosmetic composition ranges from 0.1% to 20% by weight, preferably from 0.5% to 10% by weight and more preferably still from 0.6o to 5% by weight, with respect to the total weight of the composition.

14. Use according to any one of Claims 2 to 13, **characterized in that** the anionic fixative polymer(s)/non-ionic compound(s) ratio by weight in the composition is greater than 1.

15. Use according to any one of Claims 2 to 14, **characterized in that** the cosmetic composition comprises at least one adjuvant chosen from non-ionic, anionic, cationic and amphoteric surfactant agents, additional non-ionic, anionic, cationic and amphoteric polymers, ceramides and pseudoceramides, vitamins and provitamins, including panthenol, water-soluble and fat-soluble sunscreens which are liquid or solid, solid compounds, such as pigments, pearlescent or opacifying agents, dyes, sequestering agents, plasticizing agents, solubilizing agents, acidifying agents, basifying agents, neutralizing agents, inorganic and organic thickening agents, antioxidants, hydroxy acids, solvents, penetrating agents, fragrances, buffers, dispersing agents, conditioning agents, opacifying agents and preservatives.
